# DEMANDE DE BREVET EUROPEEN

(11) **EP 1 932 511 A2**
(43) Date de publication de la demande: **18.06.2008**
(21) Numéro de dépôt: 07120377.2
(22) Date de dépôt: 09.11.2007
(51) Int. Cl.: A61K 8/365, A61K 8/368, A61Q 19/02, A61Q 19/08, A61K 8/81, A61K 8/60

(54) **Procédé de peeling à base de microgel cationique**

(30) Priorité: 30.11.2006 FR 0655198
(71) Demandeur: L'ORÉAL, 75008 Paris (FR)
(72) Inventeur: Aubrun-Sonneville, Odile, 92160, ANTONY (FR)
(74) Mandataire: Duvert, Sandra

(57) **Abrégé**

La présente invention concerne un procédé de traitement cosmétique des irrégularités visibles et/ou tactiles de la peau humaine, comprenant les étapes consistant à :
(a) appliquer topiquement sur la peau, une composition comprenant dans un milieu physiologiquement acceptable, (i) au moins un hydroxyacide choisi parmi les α-hydroxyacides, les β-hydroxyacides les α-céto-acides ; les β-céto-acides ; et leurs mélanges, (ii) au moins un polymère cationique,
b) laisser la composition au contact de la peau pendant un temps suffisant pour que la composition agisse, et
(c) éventuellement éliminer la composition par rinçage.

## Description

La présente invention se rapporte à un procédé de peeling qui met en oeuvre une composition contenant au moins un hydroxyacide et au moins un polymère cationique.

Les peelings sont un moyen bien connu pour améliorer l'aspect de surface de la peau, en particulier pour traiter les irrégularités visibles et/ou tactiles de la peau humaine, et par exemple atténuer des défauts de pigmentation tels que les lentigo actiniques ou les marques d'acné ou de varicelle, ou pour lisser les irrégularités de la texture de la peau, en particulier les rides et ridules.

Ces peelings ont pour effet d'enlever une partie de la peau à traiter (épiderme et éventuellement couche superficielle du derme), par des méthodes chimiques telles que l'application de compositions contenant des concentrations élevées d'agents stimulant la desquamation de la peau, tels que les hydroxyacides comme l'acide glycolique ou l'acide salicylique, ou encore d'autres actifs comme par exemple l'acide rétinoïque, la résorcine, l'acide trichloracétique, le phénol. Ainsi, le document US-A-6,787,148 décrit des compositions anhydres contenant un phénol et un dérivé de polyéthylène glycol.

Des concentrations croissantes en actifs et notamment en hydroxyacides permettent d'augmenter l'efficacité des produits. Toutefois, de telles concentrations conduisent à des désagréments importants à l'application et après application (rougeurs, picotements, sensation de brûlure). Ainsi, les peelings à l'acide salicylique peuvent donner lieu à des cas de salicylisme en cas de surdosage ou d'application prolongée.

Pour améliorer la tolérance des compositions de peeling à base d'hydroxyacides, il a par exemple été proposé dans le document US-A-6,787,148 d'y associer des polyéthylène glycols tels que le polyéthylène glycol 1500. Toutefois, l'introduction de tels composés nuisent aux qualités cosmétiques de la composition car ils induisent un effet collant et entraînent une mauvaise rinçabilité de la composition.

Il subsiste donc le besoin de disposer de compositions de peeling, qui soient efficaces tout en étant mieux tolérés et en ayant de bonnes propriétés cosmétiques.

La Demanderesse a trouvé de manière surprenante que l'association d'hydroxyacides et d'au moins un polymère cationique permettait d'avoir des compositions de peeling à la fois efficaces et bien tolérées, et ayant de bonnes propriétés cosmétiques.

Rien ne suggérait dans l'art antérieur que les polymères cationiques pouvaient être associés à des AHA ou BHA pour la réalisation de peelings chimiques cosmétiques à la fois performants et mieux tolérés. Certes, le document EP-A-680748 décrit des compositions contenant des hydroxyacides et des polymères cationiques mais ces compositions sont utilisées pour dépigmenter la peau et non comme peeling. Par ailleurs, le document EP-A-395282 décrit une solution acide aqueuse épaissie par des polymères cationiques, mais l'utilisation de la solution comme peeling chimique cosmétique n'est pas décrite.

La présente invention porte sur un procédé de peeling.

L'invention a donc pour objet un procédé de traitement cosmétique des irrégularités visibles et/ou tactiles de la peau humaine, comprenant les étapes consistant à :
(a) appliquer topiquement sur la peau, une composition comprenant dans un milieu physiologiquement acceptable, (i) au moins 5 % en poids d'un ou plusieurs hydroxyacides choisis parmi les α-hydroxyacides, les β-hydroxyacides les α-céto-acides ; les β-céto-acides ; et leurs mélanges, par rapport au poids total de la composition, (ii) au moins un polymère cationique réticulé,
b) laisser la composition au contact de la peau pendant un temps suffisant pour que la composition agisse, et
(c) éventuellement éliminer la composition par rinçage.

La composition est laissée au contact de la peau pendant un temps d'application qui doit être suffisant pour que la composition agisse. Ce temps variera en fonction de la concentration en hydroxyacides dans la composition, et de l'effet recherché. A titre indicatif, la composition pourra rester en contact avec la peau ou les phanères pendant une durée d'au moins 5 minutes, généralement comprise entre 5 minutes et 12 heures, de préférence entre 5 minutes et 6 heures, préférentiellement entre 5 minutes et 30 minutes. La composition pourra ou non être éliminée à l'issue de ce temps de contact. L'application pourra être quotidienne ou bi-quotidienne, ou hebdomadaire, et réitérée pendant des périodes de 2 semaines à 6 mois; cette période pouvant être prolongée ou renouvelée sans difficulté.

La composition étant destinée à une application topique, elle comprend un milieu physiologiquement acceptable. On entend par « milieu physiologiquement acceptable » un milieu compatible avec les matières kératiniques telles que la peau, les lèvres, les ongles, le cuir chevelu et/ou les cheveux. La composition est notamment une composition cosmétique ou dermatologique, plus particulièrement destinée au peeling de la peau.

Le milieu est un milieu aqueux, c'est-à-dire qu'il contient de l'eau dont la quantité est de préférence d'au moins 20 % en poids par rapport au poids total de la composition. La quantité d'eau peut aller par exemple de 20 à 95 % en poids, de préférence de 30 à 90 % en poids et mieux de 35 à 80 % en poids par rapport au poids total de la composition.

La composition a un pH qui peut aller de 0,4 à 7, de préférence de 0,5 à 5 et mieux de 1 à 4.

La composition selon l'invention est exempte de PP-14 butyl ether.

### Hydroxyacides

La composition selon l'invention contient un ou plusieurs hydroxyacides choisis parmi les α-hydroxy-acides, les β-hydroxy-acides, les α-céto-acides ; les β-céto-acides ; et leurs mélanges. Selon un mode préféré de réalisation de l'invention, les hydroxyacides sont choisis parmi les α-hydroxy-acides et les α-céto-acides ; et leurs mélanges.

On peut citer plus particulièrement comme α-hydroxy-acides, l'acide citrique, l'acide lactique, l'acide glycolique, l'acide tartrique, l'acide malique, l'acide mandélique, l'acide méthyllactique, l'acide glucuronique, l'acide pyruvique, l'acide phényllactique ; l'acide gluconique ; l'acide galacturonique, et leurs mélanges.

On peut citer plus particulièrement comme β-hydroxy-acides, l'acide salicylique et ses dérivés, en particulier ses dérivés alcoylés de formule (I) suivante ou un sel d'un tel dérivé : 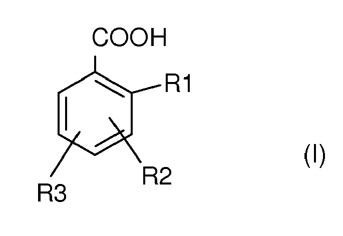 dans laquelle :
- R₁ représente un radical hydroxyle ou un ester de formule :

   -O-CO-R₄

   dans laquelle R₄ est un radical aliphatique, saturé ou insaturé, comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone,
- R₂ et R₃ indépendamment l'un de l'autre se trouvent en position 3, 4, 5 ou 6 sur le noyau benzénique et représentent indépendamment l'un de l'autre, un atome d'hydrogène ou un radical :

   -(O)ₙ-(CO)ₘ-R₅

   dans lequel n et m, indépendamment l'un de l'autre, sont chacun un nombre entier égal à 0 ou 1 ; à la condition que R₂ et R₃ ne soient pas simultanément des atomes d'hydrogène ;
- R₅ représente un atome d'hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone.

De manière préférée, le dérivé d'acide salicylique de formule (I) est tel que R₁ représente un radical hydroxyle, R₂ représente un atome d'hydrogène, R₃ est en position 5 du noyau benzénique et représente un radical -CO-R₅ où R₅ représente un radical aliphatique saturé comprenant de 3 à 15 atomes de carbone.

Selon un mode de réalisation préféré de l'invention, le dérivé d'acide salicylique de formule (I) est choisi parmi les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents et leurs mélanges. Il s'agit plus particulièrement de l'acide n-octanoyl-5-salicylique (nom INCI : Capryloyl salicylic Acid).

On peut citer plus particulièrement comme α-céto-acides, l'acide ascorbique (ou vitamine C), et les glucosides d'ascorbyle. Par "glucoside d'ascorbyle", on entend le produit de condensation du glucose, sous forme D, c'est-à-dire sous forme de glucopyrannose α ou β ou de furannose α ou β, ou sous forme L, avec l'acide ascorbique, de préférence sous forme L. On peut citer plus spécialement le 2-O-α-D-glucopyranoside d'acide L-ascorbique (Nom INCI : Ascorbyl Glucoside), disponible auprès de la société Hayashibara.

La quantité d'hydroxyacide(s) dépend du but recherché. Elle est d'au moins 5 % en poids et de préférence d'au moins 10 % en poids pour avoir un peeling efficace. Elle peut aller par exemple de 5 à 70 % en poids, de préférence de 10 à 70 % en poids, mieux de 10 à 60 % en poids, encore mieux de 10 à 50 % en poids et encore mieux de 15 à 50 % en poids par rapport au poids total de la composition.

### Polymère cationique

La composition peut contenir au moins un polymère cationique réticulé, et elle peut en contenir plusieurs. La quantité (en matière active) de polymère(s) cationique(s) peut varier selon le polymère utilisé, et elle peut aller par exemple de 0,1 à 10 % en poids, de préférence de 0,15 à 7 % en poids, et mieux de 0,2 à 5 % en poids et encore mieux de 0,5 à 5 % en poids par rapport au poids total de la composition.

Les polymères cationiques utilisés dans la composition de l'invention sont réticulés et ils peuvent être des homopolymères ou des copolymères.

Les copolymères ou homopolymères cationiques réticulés sont substantiellement solubles dans le milieu aqueux, et ils sont constitués de motifs résultant de la réaction entre (i) un monomère cationique à insaturation éthylénique ou un mélange cationique de monomères à insaturation éthylénique et (ii) un agent de réticulation à polyinsaturation éthylénique, cet agent de réticulation étant présent dans le polymère à une concentration allant de 5 ppm à 45ppm, de préférence de 10 à 40 ppm et mieux encore de 10 à 20 ppm.

Ces polymères peuvent ainsi être obtenus classiquement selon la technique dite de polymérisation en émulsion à partir des différents monomères rentrant dans leur constitution.

Les monomères à polyinsaturation éthylénique utilisés comme agents de réticulation pour la préparation de ces polymères sont de préférence choisis dans le groupe constitué par le méthylène bis-acrylamide, l'éthylène glycol di-(méth)acrylate, le di-(méth)acrylamide, le cyanométhylacrylate, le vinyloxyéthyl(méth)acrylate, ou leurs seuls métalliques. De manière préférée, il s'agit du méthylène bis-acrylamide.

Le mélange cationique de monomères peut contenir, en plus des monomères cationiques, des monomères non-ioniques.

Les monomères cationiques utilisables sont notamment les di-alkylaminoalkyl-acrylates et méthacrylates, et plus spécialement les di-alkylaminoéthyl (meth)acrylates, et leurs sels quaternaires ou acides ; les dialkyl-aminoalkyl-acrylamides ou méthacrylamides et leurs sels quaternaires ou acides comme le chlorure de méthacrylamidopropyl triméthyl ammonium. Les groupes alkyles ont par exemple de 1 à 4 atomes de carbone.

Les monomères non-ioniques utilisables en association avec les monomères cationiques sont par exemple le méthacrylamide ou l'acrylamide.

Les polymères cationiques utilisables dans l'invention sont en particulier ceux décrits dans le document EP-A-395 282.

Le polymère cationique peut aussi être un polymère obtenu par polymérisation d'un mélange de monomères comprenant au moins un monomère vinylique substitué par un groupe aminé, au moins un monomère vinylique non ionique hydrophobe, au moins un monomère vinylique comportant une chaîne alkyle et une chaîne oxyalkylénée (dit monomère associatif) et au moins un monomère vinylique comportant une chaîne oxyalkylénée (dit monomère tensioactif). Ce sont des polymères associatifs qui sont décrits dans le document US-A-2004/0052746.

Le monomère vinylique substitué par un groupe aminé peut être notamment un mono- ou di-(C1-C4)alkylamino (C1-C8)-alkyl(meth)acrylate ou (meth)acrylamide, ou un (meth)acrylate ou (meth)acrylamide hétérocyclique azoté.

Le monomère vinylique non ionique hydrophobe peut être notamment un C1-C30 alkyl ester d'acide acrylique ou d'acide méthacrylique.

Dans le monomère associatif, la chaîne alkyle peut être en particulier une chaîne en C8-C40, et le monomère vinylique associatif peut être notamment un (C8-C40)alkyl (meth)acrylate polyéthoxylé.

Le monomère vinylique comportant une chaîne oxyalkylénée est exempt de chaîne alkyle, et il peut être un composé de formules :

CH₂=CH-O(CH₂)ₐO(C₃H₆O)_{b}(C₂H₄O)CH ou

CH₂=CH CH₂O(C₃H₆O)_{d}(C₂H₄O)_{c}H

Où a est 2, 3 ou 4 ; b est un nombre entier allant de 1 à 10 ; c est un nombre entier allant de 5 à 50 ; d est un nombre entier allant de 5 à 50.

Comme exemples de polymères cationiques, on peut citer par exemple l'homopolymère de chlorure de méthacryloyléthyl triméthyl ammonium réticulé (nom INCI : POLYQUATERNIUM-37) commercialisé tel quel par la société Sigma 3V sous la dénomination Synthalen CR, ou commercialisé en dispersion dans un mélange d'esters à 50% (nom INCI: Polyquaternium-37 (and) Propylene glycol Dicaprylate/Dicaprate (and) PPG-1 Trideceth-6) par la société Ciba sous la dénomination SALCARE SC 96 ; ou commercialisé en dispersion dans une huile minérale (nom INCI : Polyquaternium-37 (and) Mineral Oil (and) PPG-1 Trideceth-6) par la société Ciba sous la dénomination SALCARE SC 95 ; le copolymère éthylacrylate/diméthylamino éthyl méthacrylate cationique en émulsion à 20 % dans l'eau (nom INCI: Polyacrylate-1 Crosspolymer) commercialisé par la société Noveon sous la dénomination CARBOPOL AQUA CC, et leurs mélanges.

Les compositions selon l'invention peuvent se présenter sous toutes les formes galéniques habituellement utilisées dans les domaines cosmétique et dermatologique, notamment sous forme de gels aqueux, de lotions, d'émulsions (E/H ou H/E ou multiple). Ces compositions sont préparées selon les méthodes usuelles. Selon un mode préféré de réalisation de l'invention, la composition se présente sous forme d'un gel aqueux, hydroalcoolique ou hydroglycolique, ou d'une solution aqueuse, hydroalcoolique ou hydroglycolique.

Quand la composition selon l'invention comporte une phase huileuse, notamment quand elle est sous forme d'émulsion, la phase huileuse contient de préférence au moins une huile, notamment une huile physiologiquement acceptable. Elle peut contenir en outre d'autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle, le stéarate d'isocétyle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Quand la composition est sous forme d'émulsion, il s'agit de préférence d'une émulsion huile-dans-eau (H/E). Les émulsions contiennent généralement au moins un émulsionnant choisi notamment parmi les émulsionnants amphotères, anioniques, cationiques ou non ioniques, utilisés seuls ou en mélange. Ce sont de préférence des émulsionnants non ioniques. Ces émulsionnants sont choisis parmi ceux classiquement utilisés dans le domaine cosmétique.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations Carbopol 1342 et Pemulen par la société Noveon ; ou les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom INCI : ammonium polyacryldimethyltauramide) ou comme le polymère en émulsion commercialisé sous la dénomination Sepigel 305 par la société Seppic (nom INCI : Polyacrylamide / C13-C14 isoparaffine / laureth-7) ; les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate/1,4-cyclohexane-diméthanol (nom INCI: Diglycol/ CHDM/I sophtalates/ SIP Copolymer) vendus sous les dénominations Eastman AQ polymer (AQ35S, AQ38S, AQ55S, AQ48 Ultra) par la société Eastman Chemical.

On peut aussi préparer des émulsions sans émulsionnants, stabilisées par des particules siliconées ou des particules d'oxyde métallique tels que TiO2 ou autres, enrobées ou non.

De façon connue, la composition de l'invention peut contenir également des adjuvants habituels dans le domaine cosmétique ou dermatologique, tels que les gélifiants hydrophiles ou lipophiles, les actifs hydrophiles ou lipophiles, les conservateurs (par exemple phenoxyethanol, et parabens), les antioxydants, les solvants, les parfums, les charges, les bactéricides, les absorbeurs d'odeur, les matières colorantes, les ajusteurs de pH (acide ou base ou tampon). Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase huileuse, dans la phase aqueuse ou solubilisés dans les tensioactifs.

La composition selon l'invention peut être exempte de tensioactifs, mais elle peut éventuellement contenir un ou plusieurs tensioactifs choisis parmi les tensioactifs non ioniques, anioniques, cationiques, amphotères ou zwitterioniques, et leurs mélanges.

Les tensioactifs non ioniques peuvent être choisis parmi les tensioactifs comportant un groupement choisi par exemple parmi les groupements polyalkylène glycol tels que notamment polyéthylène glycol ou polyopropylène glyco ; les groupements polyglycérol ; les groupements sucre (glucose, maltose, sorbitol, sorbitane éthoxylé) ; et leurs mélanges.

Comme tensioactifs non ioniques, on peut citer par exemple :
- les alkylpolyglycosides et notamment les alkylpolyglucosides (APG) ayant un groupe alkyle comportant de 6 à 16 atomes de carbone (alkyl-C6-C16 polyglucosides) et de préférence 8 à 16 atomes de carbone, comme par exemple le decylglucoside (Alkyl-C9/C11-polyglucoside (1.4) tel que le produit commercialisé sous la dénomination MYDOL 10 par la société Kao Chemicals, le produit commercialisé sous la dénomination PLANTAREN 2000 UP ou PLANTACARE 2000 UP par la société Cognis, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Cognis ; et le coco-glucoside comme le produit commercialisé sous la dénomination PLANTACARE 818/UP par la société Cognis ;
- les esters de polyéthylène glycol et d'acides comportant au moins une chaîne alkyle allant de C6 à C16, de préférence de C6 à C14, tels que le myristate de polyéthylène glycol (8 OE) comme le produit commercialisé par la société Gattefossé sous la dénomination de MIRLENE ;
- les dérivés de polyéthylène glycol et de mono-, di- et tri-glycérides d'acide comportant au moins une chaîne alkyle allant de C6 à C16, de préférence de C6 à C14, et ayant au moins deux groupes d'oxyde d'éthylène, et de préférence de 6 à 8 groupes d'oxyde d'éthylène, tels que les mono-, di- et tri-glycérides d'acide caprylique et d'acide caprique, comme celui comportant 6 groupes d'oxyde d'éthylène (nom INCI : PEG-6 caprylic/capric glycérides), commercialisé sous la dénomination SOFTIGEN 767 par la société Sasol, celui comportant 8 groupes d'oxyde d'éthylène (nom INCI : PEG-8 caprylic/capric glycérides), commercialisé sous la dénomination L.A.S. par la société Gattefosse, et celui comportant 7 groupes d'oxyde d'éthylène, commercialisé sous la dénomination CETIOL HE 810 par la société Cognis (nom INCI : PEG-7 Caprylic/Capric Glycerides) ; le mono-laurate de glycéryle oxyéthyléné (20 OE) commercialisé sous la dénomination TAGAT L 2 par la société Degussa-Goldschmidt ;
- les dérivés oxyéthylénés d'esters de sorbitane et d'acide comportant au moins une chaîne alkyle allant de C6 à C16, de préférence de C6 à C14, tels que le PEG-10 Sorbitan Laurate commercialisé sous la dénomination LIPOSORB L10 par la société Lipo Chemicals ;
- les esters de sucre comportant au moins une chaîne alkyle en C6 à C16, comme le mélange de laurate de sucrose et de dilaurate de sucrose, commercialisé par la société Mitsubishi Chemical sous la dénomination SURFHOPE SE COSME C-1216 ;
- les esters de polyglycérol et d'acide comportant au moins une chaîne alkyle en C6 à C16, comme le mono-laurate de polyglycéryle (10 moles de glycéryle) (nom INCI : Polyglyceryl-10 Laurate) commercialisé par la société Sakamoto Yakuhin sous la dénomination S FACE L-1 001 ;
- les éthers de polyglycérol comme le polyglycérol-3 hydroxylauryl éther produit par Chimex sous la dénomination Chimexane NF ;
- les éthers de polyéthylène glycol et d'alcools gras comportant une chaîne alkyle de C8 à C30, tels que les éthers d'alcool cétylique, d'alcool stéarylique, d'alcool cétéarylique, d'alcool laurique,
- Et leurs mélanges.

Comme tensioactifs anioniques, on peut citer par exemple :
- les alkylsulfates, les alkyl éther sulfates et leurs sels, notamment leurs sels de sodium, comme le lauryl éther sulfate de sodium tel que le produit commercialisé sous la dénomination TEXAPON AOS 225 UP par la société Cognis ;
- les monoalkyl- et dialkylesters d'acide phosphorique et leurs sels, tels que par exemple le mono- et dilauryl phosphate de sodium, le mono- et dilauryl phosphate de potassium, le mono- et dilauryl phosphate de triéthanolamine, le mono- et dimyristyl phosphate de sodium, le mono- et dimyristyl phosphate de potassium, le mono- et dimyristyl phosphate de diéthanolamine, le mono- et dimyristyl phosphate de triéthanolamine ;
- les dérivés des aminoacides, notamment les sels alcalins d'aminoacides, tels que :
   - les acylsarcosinates comme le Lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN ® par la société Nikkol.
   - lesacyl alaninates comme le N-lauroyl-N methyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-methyl alanine triéthanolamine commercialisé sous la dénomination ALANONE ALTA ® par la société Kawaken.
   - Les acylglutamates comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
   - les acylglycinates comme le N-cocoyl glycinate de sodium commercialisé sous les dénominations AMILITE GCS-12® et AMILITE GCK 12 par la société Ajinomoto.
- les alkyl éther carboxylates notamment ceux de formule : 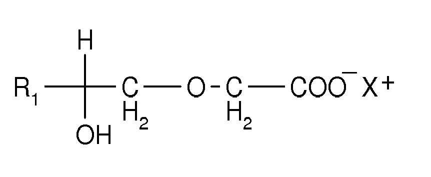 dans laquelle :
   R1 désigne plus particulièrement un radical alkyle saturé ou insaturé, linéaire ou ramifié comportant de 8 à 16 atomes de carbone,
   X désigne l'hydrogène ou un cation minéral ou organique tel que ceux issus d'un métal alcalin (par exemple Na+, K+), NH4+, les ammoniums issus des aminoacides basiques tels que la lysine, l'arginine, la sarcosine, l'ornithine, la citrulline ou bien encore des amino-alcools tels que la monoéthanolamine, la diéthanolamine, la triéthanolamine, la glucamine, la N-méthyl glucamine, l'amino-3 propanediol-1,2. Des acides hydroxy-2 alkyl éther carboxyliques préférés sont des composés de formule (I) dans laquelle R1 désigne plus particulièrement un mélange de radicaux en C8-C16, notamment dérivés de coprah. Parmi les tensioactifs de formule (I), on peut citer en particulier le produit commercialisé sous la dénomination BEAULIGHT SHAA par la société SANYO ;
- et leurs mélanges.

Les tensioactifs cationiques utilisables selon la présente invention peuvent être notamment les sels d'amines primaires, secondaires ou tertiaires, comportant une chaîne alkyle en C6 à C16, éventuellement polyoxyalkylénées ; les sels d'ammonium quaternaire ; les dérivés d'imidazoline ou les oxydes d'amines à caractère cationique.

Dans les sels d'ammonium quaternaire, l'anion est de préférence un halogénure (chlorure, bromure ou iodure) ou un alkylsulfate plus particulièrement un méthylsulfate. On peut cependant utiliser des sels d'ammonium quaternaire où l'anion est un méthanesulfonate, un phosphate, un nitrate, un tosylate, un anion dérivé d'acide organique tel que l'acétate ou le lactate ou tout autre anion compatible avec l'ammonium à fonction ester. L'anion est encore plus particulièrement le chlorure ou le méthylsulfate.

Parmi les sels d'ammonium quaternaire, on peut citer notamment :
- les chlorures de tétraalkylammonium comme, par exemple, les chlorures de dialkyldiméthylammonium ou d'alkyltriméthylammonium, dans lesquels le radical alkyle comporte environ de 6 à 16 atomes de carbone, comme par exemple le chlorure de dodecyl triméthyl ammonium commercialisé sous la dénomination ARQUAD 12-50 par la société Akzo Nobel, ou le chlorure de cétyl triméthyl ammonium commercialisé sous la dénomination DEHYQUART A OR par la société Cognis ;
- les sels d'ammonium quaternaire contenant au moins une fonction ester, comme par exemple le méthosulfate de dicocoyléthyl hydroxyéthyl méthyl ammonium commercialisé sous la dénomination DEHYQUART L 80 par la société Cognis ;
- les sels d'ammonium quaternaire contenant un motif sucre, par exemple un motif glucose, fructose, saccharose, comme par exemple le chlorure de Butyldimoniumhydroxypropyl Laurylglucosides (nom INCI : Butyldimoniumhydroxypropyl Laurylglucosides Chloride) commercialisé sous la dénomination Colonial SugaQuat TM-1212 par la société Colonial Chemical Inc, le chlorure de Lauryl Methyl Gluceth-10 Hydroxypropyldimonium (nom INCI :Lauryl Methyl Gluceth-10 Hydroxypropyldimonium Chloride) commercialisé sous la dénomination GLUCQUAT 125 par la société Noveon.

Les tensioactifs amphotères et zwitterioniques peuvent être choisis par exemple parmi les dérivés de bétaïne et notamment les dérivés alkylés de bétaïne, les alkylamidopropylbétaïnes, les alkylamphoacétates, les hydroxylsultaines, et leurs mélanges.

Comme dérivés de bétaïnes, on peut citer notamment les alkylbétal̂nes comportant un groupe alkyle en C6-C16, et plus particulièrement en C6-C14, et leurs dérivés oxyéthylénés de ces alkylbétaïnes, par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER(10 OE)BETAINE® par la société Shin Nihon Rica, la stéarylbétaïne oxyéthylénée (10 OE) comme le produit commercialisé sous la dénomination STEARYLETHER(10 OE)BETAINE® par la société Shin Nihon Rica

Comme alkylamidopropylbétaïnes, on peut citer par exemple les alkyl-C6-C16-amidopropylbétaïnes tels que la cocamidopropyl betaine commercialisé par exemple sous la dénomination VELVETEX BK 35® par Cognis ou encore le undecylenamidopropyl betaïne commercialisé par exemple sous la dénomination AMPHORAM U® par Ceca.

Comme alkylamphoacétates, on peut citer par exemple les alkyl-C6-C16-amphoacétates tels que le N-cocoyl-N-carboxyméthoxyéthyl-N-carboxyméthyl-éthylènediamine N-di-sodique (nom INCI : disodium cocamphodiacetate) comme le produit commercialisé sous la dénomination MIRANOL C2M CONCENTRE NP® par la société Rhodia Chimie, et le N-cocoyl-N-hydroxyéthyl-N-carboxyméthyl-éthylènediamine N-sodique (nom INCI : sodium cocamphoacetate).

La quantité de tensioactifs (en matière active) peut aller par exemple de 0,1 à 70 % en poids, de préférence de 0,5 à 60 % en poids, mieux de 1 à 50 % en poids et encore mieux de 1 à 30 % en poids par rapport au poids total de la composition.

Bien entendu, l'homme de l'art veillera à choisir le ou les éventuels additifs à ajouter à la composition selon l'invention et leurs quantités, de manière telle que les propriétés avantageuses attachées intrinsèquement à la composition conforme à l'invention ne soient pas, ou substantiellement pas, altérées par l'addition envisagée.

Selon un mode particulier de réalisation de l'invention, la composition contient au moins un polymère hydrophile c'est-à-dire hydrosoluble ou hydrodispersible. Comme polymères hydrophiles, on peut citer en particulier:
- les dérivés cellulosiques (carboxyméthylcellulose, hydroxyéthylcellulose, hydroxypropylméthylcellulose) ;
- les gommes naturelles telles que les gommes de xanthane, de guar, de caroube, les carraghénanes ;
- les polymères polycarboxyvinyliques du type Carbomer, tels que ceux vendus par la société Goodrich sous les dénominations Carbopol 940, 951, 980, ou par la société 3V-Sigma sous la dénomination Synthalen K ou Synthalen L ;
- les copolymères acryliques tels que les copolymères d'acrylates/alkylacrylates vendus sous les dénominations Pemulen par la société Goodrich ;
- les polyacrylamides et les copolymères d'acrylamide, indiqués ci-dessus, tels que le produit vendu sous le nom de SEPIGEL 305 par la société Seppic, le produit vendu sous le nom de HOSTACERIN AMPS par la société Clariant ou les copolymères vendus sous les dénominations ARISTOFLEX par la société Clariant.

La quantité de polymère(s) hydrophile(s) peut aller par exemple de 0,01 à 5 % en poids, de préférence de 0,05 à 5 % en poids et mieux de 0,1 à 3 % en poids par rapport au poids total de la composition.

Par ailleurs, la composition peut contenir en outre au moins un composé hydroxylé hydrosoluble choisi parmi les alcools monohydriques en C2-C6 et de préférence en C2-C4 tels que l'éthanol et l'isopropanol, et les polyols comprenant de 1 à 3 atomes de carbone, tels que la glycérine, le propylène glycol, le butylène glycol, le dipropylène glycol, l'isopropylène glycol ; et leurs mélanges. La quantité de composé(s) hydroxylé(s) peut aller par exemple de 0,1 à 70 % en poids, mieux de 1 à 65 % en poids et encore mieux de 1 à 60 % en poids par rapport au poids total de la composition.

Quand la composition est hydroalcoolique, elle contient de l'eau et au moins un alcool monohydrique, et quand la composition est hydroglycolique, elle contient de l'eau et au moins un polyol. Elle peut bien sûr contenir à la fois des alcools et des polyols.

La composition peut contenir aussi tout actif approprié autre que les hydroxyacides cités ci-dessus, tel que par exemple l'urée et ses dérivés hydroxylés comme le N-(2-hydroxyéthyl)-urée commercialisé sous la dénomination hydrovance par la société National Sstarch ; l'acide hyaluronique ; les polymères hydratants comme les polymères acryliques à groupement phosphorylcholine tels que :
- le poly-2-(méthacryloyloxyéthyl)phosphorylcholine à 40 % dans un mélange eau/butanediol (5% de butanediol) vendu sous la dénomination LIPIDURE HM par la société Nippon Oils and Fats (nom INCI : polyphosphorylcholine glycol acrylate (and) butylene glycol).
- le copolymère de 2-(méthacryloyloxyéthyl) phosphorylcholine/méthacrylate de butyle (90/10) à 5 % en solution dans l'eau vendu sous la dénomination LIPIDURE PMB par la société Nippon Oils and Fats ; (nom INCI : Polyquaternium-51).
- le copolymère de 2-(méthacryloyloxyéthyl)phosphorylcholine/chlorure de 2-hydroxy-3-méthacryloyloxypropyltriméthylammonium à 5% en solution dans l'eau, vendu sous la dénomination LIPIDURE-C par la société Nippon Oils and Fats.
- le terpolymère de 2-(méthacryloyloxyéthyl) phosphorylcholine/méthacrylate de butyle/méthacrylate de sodium à 5 % en solution dans l'eau, vendu sous la dénomination LIPIDURE-A par la société Nippon Oils and Fats.
- les copolymères de 2-(méthacryloyloxyéthyl) phosphorylcholine/méthacrylate de stéaryle vendus sous les dénominations LIPIDURE-S, LIPIDURE-NR, LIPIDURE-NA par la société Nippon Oils and Fats (nom INCI : Polyquaternium-61).

La composition peut contenir aussi d'autres actifs tels que des vitamines comme les vitamines A, C, E, B3, B5, K et leurs dérivés, notamment leurs esters, et des séquestrants comme l'EDTA.

Comme charges, la composition de l'invention peut contenir par exemple des particules minérales telles que les argiles, les silices, les oxydes métalliques tels que le dioxyde de titane ou l'oxyde de zinc, le mica, et/ou des charges organiques telles que les particules de polyamide (Nylon) et notamment celles vendues sous les dénominations ORGASOL par la société Atochem ; les latex ; les poudres de polyéthylène ; les microsphères à base de copolymères acryliques, telles que celles en copolymère diméthacrylate d'éthylène glycol/methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; et leurs mélanges. La quantité de charge(s) peut aller par exemple de 0,05 à 20 % en poids et mieux 0,1 à 10 % en poids par rapport au poids total de la composition.

Comme indiqué précédemment, cette composition est destinée à être mise en oeuvre dans un procédé de peeling visant à atténuer les irrégularités visibles et/ou tactiles de la peau, et en particulier à atténuer les rides et ridules et/ou les taches pigmentaires et/ou les cicatrices, en particulier les marques d'acné- et/ou à désobstruer les pores de la peau et donner plus d'éclat à la peau. La composition peut donc être appliquée en particulier sur le visage et/ou le cou et/ou le décolleté et/ou les mains et/ou le dos.

Pour optimiser ses effets, le procédé de peeling selon l'invention comprend de préférence des étapes additionnelles de préparation de la peau au peeling et/ou de soin de la peau après peeling à l'aide de compositions renfermant de plus faibles quantités d'actifs que la composition de peeling décrite précédemment.

La mise en oeuvre de l'étape préliminaire ci-dessus permet en outre de dépister une éventuelle allergie aux actifs et d'améliorer l'efficacité et l'homogénéité du peeling.

Ainsi, selon un mode de réalisation particulier, le procédé selon l'invention comprend, outre les étapes mentionnées précédemment :
- une étape préliminaire d'application sur la peau d'une composition contenant, dans un milieu physiologiquement acceptable, soit de 0,1 à 10 % en poids d'un ou plusieurs polymères cationiques par rapport au poids total de la composition, soit 3 à 10 % en poids d'un ou plusieurs hydroxyacides par rapport au poids total de la composition, soit 3 à 10 % en poids d'un mélange d'un ou plusieurs polymères cationiques et d'un ou plusieurs hydroxyacides par rapport au poids total de la composition, avant la mise en oeuvre de l'étape (a), et/ou
- une étape supplémentaire d'application sur la peau d'une composition contenant, dans un milieu physiologiquement acceptable, soit de 10 à 50 % en poids d'un ou plusieurs polymères cationiques, par rapport au poids total de la composition, soit de 10 à 50 % d'un ou plusieurs hydroxyacides par rapport au poids total de la composition, soit de 10 à 50 % d'un mélange d'un ou plusieurs polymères cationiques et d'un ou plusieurs hydroxyacides par rapport au poids total de la composition, après la mise en oeuvre de l'étape (c).

Les compositions utilisées dans ces étapes préliminaire et supplémentaire peuvent être appliquées matin et soir, par exemple, éventuellement en association avec une composition destinée à protéger la peau contre les effets des UV. La composition de pré-traitement peut être appliquée pendant une à quatre semaines et la composition de post-traitement pendant un jour à huit semaines, par exemple.

Le procédé selon l'invention, y compris les étapes préliminaire et supplémentaire éventuelles, peut être mis en oeuvre une seule fois ou renouvelé jusqu'à cinq fois, si nécessaire, les séances de peeling étant de préférence espacées de une à huit semaines.

L'invention sera maintenant illustrée par les exemples non limitatifs suivants. Dans ces exemples, les quantités sont indiquées en pourcentage en poids, et ce sont des quantités de matière active. Selon les cas, les noms indiqués sont les noms chimiques ou les noms INCI.

### Exemples 1 à 5 selon l'invention

| | Exemple 1 | Exemple 2 | Exemple 3 | Exemple 4 | Exemple 5 |
|---|---|---|---|---|---|
| Polyacrylate-1 crosspolymer (1) | 2 | 2 | - | - | 3 |
| Polyquaternium-37 (and) mineral oil (and) PPG-1 tridéceth-6 (2) | | | 2 | 2 | - |
| Acide glycolique | 30 | 50 | 20 | 50 | - |
| Acide lactique | - | - | - | - | 30 |
| Eau | Qsp 100 | Qsp 100 | Qsp 100 | Qsp 100 | Qsp 100 |
| pH | 1,2 | 0,7 | 1,5 | 0,46 | Non mesuré |

| | | | | | |
|---|---|---|---|---|---|
| (1) Carbopol Aqua CC de Novéon qui est à 30% en matière active. Pour avoir 2% de matière active, on met 6,67% de matière première, et pour en avoir 3%, on met 10% de matière première. (2) Salcare SC95 de Ciba, qui est à 50% en matière active. Pour avoir 2% de matière active, on met 4% de matière première. | | | | | |

Mode opératoire : On a dispersé dans l'eau le polymère cationique, éventuellement en chauffant un peu, puis on a ajouté les AHA sous agitation.

La composition obtenue peut être utilisée pour le peeling de la peau, et elle est aussi efficace qu'une composition contenant une quantité équivalente d'AHA tout en étant moins agressive, comme le montrent les tests décrits ci-dessous.

### Tolérance in vitro des produits de peeling

La tolérance des produits a été évaluée in vitro sur épidermes reconstruits (EpiSkin^{SM}).

Le protocole utilisé (EpiPeel) permet la mise en place d'une stratégie d'évaluation d'actifs en formulation simplex, fortement concentrés. Ce protocole utilise la complémentarité de deux tests réalisés in vitro sur épidermes reconstruits : test prédictif de la corrosion (validé en Europe comme test de remplacement du modèle animal, TG 431), et test prédictif du potentiel irritant des produits chimiques. EpiPeel permet de définir une gradation « standard » des effets sur 5 niveaux d'activité. Son orientation actuelle vise l'évaluation de la tolérance cutanée de produits formulés ou non formulés à de fortes concentrations dont les temps de contacts recommandés sont de courte durée et réalisés sous contrôle.

**Modèle prédictif EpiPeel**

| | Niveau 1 | Niveau 2 | Niveau 3 | Niveau 4 | Niveau 5 |
|---|---|---|---|---|---|
| Corrosion EU | R35 | R34 | R34 | NC | NC |
| Corrosion UN | Class 1 | Class II | Class III | NC | NC |
| Tolérance | | | | Irritant | Non Irritant |

| | | | | | |
|---|---|---|---|---|---|
| NC : non corrosive | | | | | |

Modèle Prédictif de la corrosion (OECD guideline TG 431) :
R35/classe I : corrosif sévère
   % viabilité < 35% après 3 min de contact
R34/classe II : corrosif
   % viabilité ≥35% après 3 min de contact et < 35% après 1 heure de contact
R34/classe III : corrosif
   % viabilité ≥35% après 1 heure de contact et < 35% après 4 heures de contact

UN: United Nations Classification EU: European Union Classification

Le potentiel de corrosion de l'exemple 1, a été trouvé R34/classe III, alors que celui d'une solution d'acide glycolique à 30% au même pH a été trouvé comme R34/classe II, donc cette solution est plus corrosive que l'exemple 1 selon l'invention.

### Test d'efficacité peeling in vitro

Un test in vitro a été réalisé pour déterminer l'efficacité de l'exemple 1 selon l'invention par rapport à une composition sans polymère cationique, comprenant 30 % d'acide glycolique et 70 % d'eau. Le nombre de cornéocytes libérés après application de la composition selon l'invention a été d'environ 80/µl pour les deux supports. Leur efficacité peeling in vitro est par conséquent équivalente.

Par conséquent, la composition selon l'invention présente l'avantage d'être mieux tolérée pour une efficacité identique.

## Revendications

1. Procédé de traitement cosmétique des irrégularités visibles et/ou tactiles de la peau humaine, comprenant les étapes consistant à :
(a) appliquer topiquement sur la peau, une composition comprenant dans un milieu physiologiquement acceptable, (i) au moins 5 % en poids d'un ou plusieurs hydroxyacides choisis parmi les α-hydroxyacides, les β-hydroxyacides les α-céto-acides ; les β-céto-acides ; et leurs mélanges, par rapport au poids total de la composition, (ii) au moins un polymère cationique réticulé,
b) laisser la composition au contact de la peau pendant un temps suffisant pour que la composition agisse, et
(c) éventuellement éliminer la composition par rinçage.

2. Procédé selon la revendication 1, **caractérisé en ce que** la composition contient au moins 20 % en poids d'eau par rapport au poids total de la composition.

3. Procédé selon la revendication 1 ou 2, **caractérisé en ce que** les α-hydroxyacides sont choisis parmi l'acide citrique, l'acide lactique, l'acide glycolique, l'acide tartrique, l'acide malique, l'acide mandélique, l'acide méthyllactique, l'acide glucuronique, l'acide pyruvique, l'acide phényllactique ; l'acide gluconique ; l'acide galacturonique, et leurs mélanges.

4. Procédé selon l'une quelconque des revendications précédentes, **caractérisé en ce que** les β-hydroxy-acides sont choisis parmi l'acide salicylique et ses dérivés alcoylés de formule (I) suivante ou un sel d'un tel dérivé : 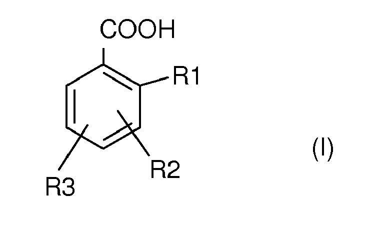 dans laquelle :
- R₁ représente un radical hydroxyle ou un ester de formule :
-O-CO-R₄
dans laquelle R₄ est un radical aliphatique, saturé ou insaturé, comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone,
- R₂ et R₃ indépendamment l'un de l'autre se trouvent en position 3, 4, 5 ou 6 sur le noyau benzénique et représentent indépendamment l'un de l'autre, un atome d'hydrogène ou un radical :
-(O)ₙ-(CO)ₘ-R₅
dans lequel n et m, indépendamment l'un de l'autre, sont chacun un nombre entier égal à 0 ou 1 ; à la condition que R₂ et R₃ ne soient pas simultanément des atomes d'hydrogène ;
- R₅ représente un atome d'hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone.

5. Procédé selon l'une quelconque des revendications précédentes, **caractérisé en ce que** les α-céto-acides sont choisis parmi l'acide ascorbique et les glucosides d'ascorbyle.

6. Procédé selon l'une quelconque des revendications précédentes, **caractérisé en ce que** la quantité d'hydroxyacide(s) va de 5 à 70 % en poids et de préférence de 10 à 70 % en poids par rapport au poids total de la composition.

7. Procédé selon l'une quelconque des revendications précédentes, **caractérisé en ce que** la quantité de polymère(s) cationique(s) va de 0.1 à 10% en poids et de préférence de 0.2 à 5% en poids par rapport au poids total de la composition.

8. Procédé selon l'une quelconque des revendications précédentes, **caractérisé en ce que** le polymère cationique est un polymère cationique réticulé constitué de motifs résultant de la réaction entre (i) un monomère cationique à insaturation éthylénique ou un mélange cationique de monomères à insaturation éthylénique et (ii) un agent de réticulation à polyinsaturation éthylénique.

9. Procédé selon l'une quelconque des revendications 1 à 7, **caractérisé en ce que** le polymère cationique est obtenu par polymérisation d'un mélange de monomères comprenant au moins un monomère vinylique substitué par un groupe aminé, au moins un monomère vinylique non ionique hydrophobe, au moins un monomère vinylique comportant une chaîne alkyle et une chaîne oxyalkylénée et au moins un monomère vinylique comportant une chaîne oxyalkylénée.

10. Procédé selon l'une quelconque des revendications 1 à 9, **caractérisé en ce que** le polymère cationique est choisi parmi l'homopolymère de chlorure de méthacryloyléthyl triméthyl ammonium réticulé, le copolymère éthylacrylate/diméthylamino éthyl méthacrylate cationique, et leurs mélanges.

11. Procédé selon l'une quelconque des revendications précédentes, **caractérisé en ce que** la composition est laissée au contact de la peau pendant une durée comprise entre 5 minutes et 12 heures.

12. Procédé selon l'une quelconque des revendications précédentes, **caractérisé en ce qu'**il comprend en outre une étape préliminaire d'application sur la peau, d'une composition contenant, dans un milieu physiologiquement acceptable, soit de 0,1 à 10 % % en poids d'un ou plusieurs polymères cationiques par rapport au poids total de la composition, soit de 3 à 10 % d'un ou plusieurs hydroxyacides par rapport au poids total de la composition, soit de 3 à 10 % d'un mélange d'un ou plusieurs polymères cationiques et d'un ou plusieurs hydroxyacides par rapport au poids total de la composition, avant la mise en oeuvre de l'étape (a).

13. Procédé selon l'une quelconque des revendications précédentes, **caractérisé en ce qu'**il comprend en outre une étape supplémentaire d'application sur la peau d'une composition contenant, dans un milieu physiologiquement acceptable, soit de 10 à 50 % en poids d'un ou plusieurs polymères cationiques par rapport au poids total de la composition, soit de 10 à 50 % en poids d'un ou plusieurs hydroxyacides par rapport au poids total de la composition, soit de 10 à 50 % d'un mélange d'un ou plusieurs polymères cationiques et d'un ou plusieurs hydroxyacides par rapport au poids total de la composition, après la mise en oeuvre de l'étape (c).

14. Procédé selon l'une quelconque des revendications précédentes, **caractérisé en ce qu'**il est destiné à atténuer les rides et ridules et/ou les taches pigmentaires et/ou les marques d'acné et/ou à désobstruer les pores de la peau.

15. Procédé selon l'une quelconque des revendications précédentes, **caractérisé en ce que** la composition est appliquée sur le visage et/ou le cou et/ou le décolleté et/ou les mains et/ou le dos.
